# EUROPÄISCHE PATENTANMELDUNG

(11) **EP 1 905 767 A1**
(43) Veröffentlichungstag der Anmeldung: **02.04.2008**
(21) Anmeldenummer: 06020345.2
(22) Anmeldetag: 28.09.2006
(51) Int. Cl.: C07D 317/12, C07D 317/20, C07D 317/22, C07D 319/06

(54) **Verfahren zur Herstellung von Glycerinacetalen**

(71) Anmelder: Cognis IP Management GmbH, 40589 Düsseldorf (DE)
(72) Erfinder: Sato, Setsuo, BR-12243-260 São José dos Campos (ES); Araújo, Alexssander Shigueru, BR-12237-000 São José dos Campos (ES); Miyano, Mirian, BR-12246-150 São José dos Campos (ES)
(74) Vertreter: Reinhardt, Jürgen

(57) **Zusammenfassung**

Vorgeschlagen wird ein Verfahren zur Herstellung von Glycerinacetalen von Aldehyden ausgewählt aus der Gruppe von Isobutyraldehyd, 2-Ethylhexylaldehyd, Furfurylaldehyd und Benzaldehyd, wobei man Glycerin mit einem oder mehreren der genannten Aldehyde in Abwesenheit von organischen Lösungsmitteln und in Gegenwart von Phosphorsäure als Katalysator umsetzt, wobei man ein molares Verhältnis von Glycerin zu Aldehyd(en) im Bereich von 1 : 1 bis 1 : 1,5 einstellt.

## Beschreibung

### Gebiet der Erfindung

Die Erfindung betrifft ein verbessertes Verfahren zur Herstellung von speziellen Glycerinacetalen. Das Verfahren zeichnet sich dadurch aus, daß es lösungsmittelfrei arbeitet, Produkte hoher Reinheit liefert, und bei mäßigen Reaktionszeiten ausgezeichnete Ausbeuten aufweist.

### Stand der Technik

Acetale und Ketale des Glycerins sind bekannt. Sie werden im Allgemeinen durch Umsetzung von Glycerin mit Aldehyden bzw. Ketonen in Gegenwart von organischen Lösungsmitteln und metallorganische Katalysatoren wie Dibutylzinndichlorid bzw. sauren Katalysatoren wie p-Toluolsulfonsäure hergestellt.

### Beschreibung der Erfindung

Die Aufgabe der vorliegenden Erfindung bestand darin, ein verbessertes Verfahren zur Herstellung von Glycerinacetalen des Isobutyraldehyds und/oder des 2-Ethylhexylaldehyds und/oder des Furfurylaldehyds und/oder des Benzaldehyds bereitzustellen, das sich dadurch auszeichnet, dass es lösungsmittelfrei arbeitet, Produkte hoher Reinheit liefert, und bei mäßigen Reaktionszeiten ausgezeichnete Ausbeuten aufweist. Die erhaltenen Produkte sollten farblos sein, ohne dass es hierzu spezieller weiterer Aufarbeitungsschritte bedarf.

Die herzustellenden Glycerinacetale des Isobutyraldehyds, 2-Ethylhexylaldehyds, Furfurylaldehyds und Benzaldehyds weisen folgende Formeln auf:

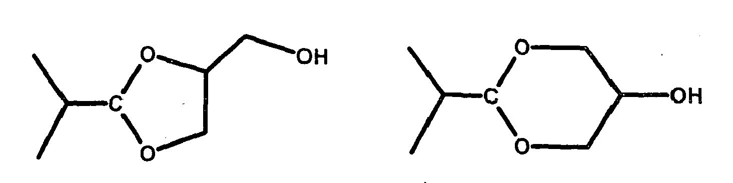

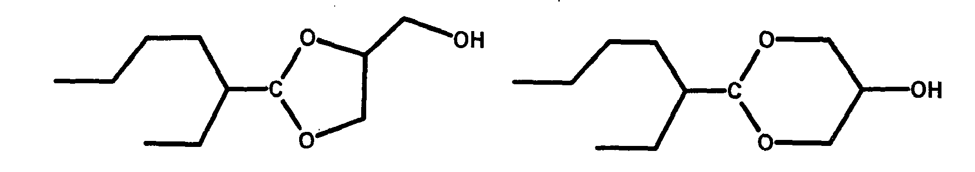

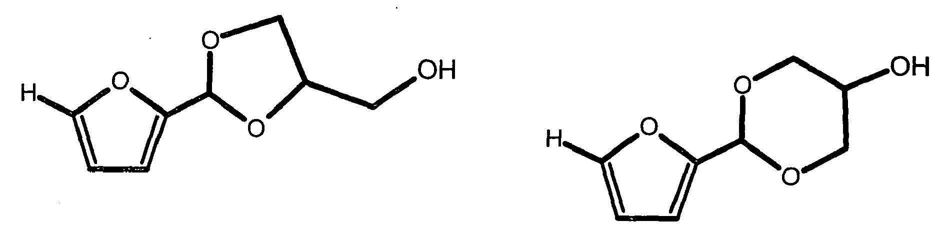

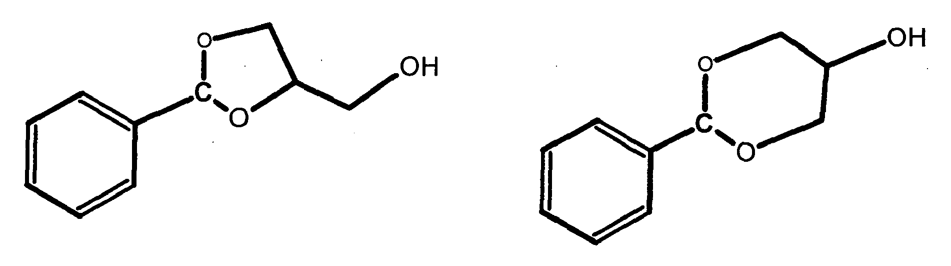

Die genannte Aufgabe wurde durch das Verfahren der vorliegenden Erfindung gelöst.

Die essentiellen Verfahrensschritte des erfindungsgemäßen Verfahrens sind wie folgt:
1. Man setzt Glycerin mit ein oder mehreren Aldehyden ausgewählt aus der Gruppe Isobutyraldehyd, 2-Ethylhexylaldehyd, Furfurylaldehyd und Benzaldehyd um und zwar
2. in Abwesenheit von organischen Lösungsmitteln und
3. in Gegenwart von Phosphorsäure als Katalysator,
4. wobei man ein molares Verhältnis von Glycerin zu Aldehyd(en) im Bereich von 1 : 1 bis 1 : 1,5 einstellt (wobei ein Verhältnis von 1 : 1,2 und insbesondere 1 : 1,1 besonders bevorzugt ist).

Gegenstand der Erfindung ist ein Verfahren zur Herstellung von Glycerinacetalen von Aldehyden ausgewählt aus der Gruppe von Isobutyraldehyd, 2-Ethylhexylaldehyd, Furfurylaldehyd und Benzaldehyd wobei man Glycerin mit ein oder mehreren der genannten Aldehyde in Abwesenheit von organischen Lösungsmitteln und in Gegenwart von Phosphorsäure als Katalysator umsetzt, wobei man ein molares Verhältnis von Glycerin zu Aldehyd(en) im Bereich von 1 : 1 bis 1 : 1,5 einstellt.

Der Klarheit halber sei festgestellt: Wenn vom molaren Verhältnis von Glycerin zu Aldehyden die Rede ist, dann bezieht sich die Singularform "Aldehyd" darauf, dass entweder Isobutyraldehyd oder 2-Ethylhexylaldehyd oder Furfurylaldehyd oder Benzaldehyd eingesetzt wird und die Pluralform "Aldehyden" darauf, dass ein oder mehrere der genannten Aldehyde eingesetzt werden. Bei dem Glycerin-Aldehyd-Verhältnis geht es also stets um das molare Verhältnis von Glycerin zur Gesamtheit an Aldehyd(en).

Nach dem erfindungsgemäßen Verfahren fallen die gewünschten Acetale in hoher Ausbeute (mindestens 95% bezogen auf eingesetztes Glycerin) an. Die Acetale weisen eine gaschromatographisch bestimmte Reinheit von mindestens 95% (Flächenprozent GC) auf, ohne dass es spezieller Reinigungsschritte wie Filtration oder zusätzliche Destillation bedürfte. Die erhaltenen Produkte sind außerdem farblos. Letzteres ist nicht trivial - so werden nach Untersuchungen der Anmelderin dunkelfarbige Produkte erhalten, wenn anstelle der Phosphorsäure als Katalysator p-Toluolsulfonsäure als Katalysator eingesetzt wird.

Vorzugsweise stellt man ein molares Verhältnis von Glycerin zu Aldehyden von etwa 1 : 1,1 ein.

Glycerin kann in dem erfindungsgemäßen Verfahren in reiner Form oder mit einem Wassergehalt von bis zu 20 Gew.-% eingesetzt werden. Letztgenannte Rohstoffe (also wasserhaltige Glycerine) stehen beispielsweise aus dem Biodiesel-Prozess zur Verfügung.

Das erfindungsgemäße Verfahren zeichnet sich durch rasche bis mäßige Reaktionszeiten aus. In der Regel reichen 3 bis 6 Stunden für einen nahezu quantitativen Umsatz aus.

Der eingesetzte Katalysator (Phosphorsäure) wird vorzugsweise in einer Menge von 0,1 bis 0,8 mol-% (und vorzugsweise 0,2 bis 0,6 mol-%) - bezogen auf Glycerin - eingesetzt. wird vorzugsweise bei Temperaturen im Bereich von 40 bis 150 °C und vorzugsweise von 70 bis 130 °C durchgeführt. Das Reaktionswasser wird dabei in einer Ausführungsform kontinuierlich aus dem Reaktionsgemisch entfernt, vorzugsweise durch eine Vorrichtung vom Dean Stark Typ.

Vorzugsweise wird die Reaktion unter Inertgas, etwa Stickstoff durchgeführt.

Bei dem erfindungsgemäßen Verfahren fallen die 5- und 6-Ringe der jeweils korrespondierenden Acetale (siehe die obigen Formeln) in einem Verhältnis von etwa 80 : 20 an.

Ein weiterer Erfindungsgegenstand ist die Verwendung der durch das erfindungsgemäße Verfahren erhältlichen Glycerinacetale als Lösungsmittel, Verdünnungsmittel, Emollient, Plasticizer (=Weichmacher für Kunststoffe) und Netzmittel für die unterschiedlichsten Anwendungsbereiche beispielsweise für Kosmetik, Textiltechnik, Ledertechnik, Schmiermittel zur Metallbearbeitung, Agrochemikalien, Kunststoffe, Gummi- und Kautschukindustrie, usw. Darüber hinaus lassen sich die nach dem erfindungsgemäßen Verfahren erhältlichen Glycerinacetale als Ausgangssubstanzen für chemische Synthesen verwenden, insbesondere solchen, bei denen man die Glycerinacetale mit solchen Reaktionspartnern umsetzt, die zu Reaktionen mit OH-Gruppen befähigt sind.

Bezüglich der Eignung der durch das erfindungsgemäße Verfahren erhältlichen Glycerinacetale als Lösungsmittel sei insbesondere hervorgehoben die Eignung als Lösungsmittel für Polymere und Harze, insbesondere für Alkyd- und Polyesterharze, feste Polyamide, flüssige Epoxidharzhärter, Polyurethane, Nitrozellulose, Maleinsäurebasierte Harze. Hierbei können klassische Lösungsmittel ganz oder teilweise ersetzt werden.

### Beispiele

### Beispiel 1

Ein 50-Liter-Vielzweckreaktor mit mechanischem Rührer und aufgesetzter Destillationsvorrichtung mit Wasserabscheider und N₂-Zufuhr wurde bei 20 °C unter Stickstoffatmosphäre mit 10195g (110,8 mol) Glycerin, 40,0g (0,35 mol) Phosphorsäure (85%-ig in Wasser) und 9445g (131 mol) of iso-Butyraldehyd (der Überschuß dient im wesentlichen dazu, das Totvolumen des Wasserabscheiders zu füllen) beschickt.
Die Temperatur wurde innerhalb von 3 Stunden auf 130 °C erhöht. Während der Reaktion wurde gebildetes Reaktionswasser kontinuierlich ausgekreist. Die Reaktion war nach 2 Stunden bei 130 °C beendet. Überschüssiger iso-Butyraldehyd wurde mit einem Stickstoffstrom ausgetragen, wobei 730 g of iso-Butyraldehyd zurückgewonnen wurden. Die Menge des ausgetragenen Wassers betrug 2200g. Das Zielprodukt (Glycerinacetal von iso-Butyraldehyd) wurde in einer Menge von 16720g erhalten. Es wurde wie folgt charakterisiert:

| | |
|---|---|
| Ausbeute | 97% |
| APHA-Farbe | 30 |
| Dichte (bei 25°C) g/cm³ | 1,048 |
| Brechungsindex (bei 20°C) | 1,447 |
| Gefrierpunkt (°C) | <-50C |
| Löslichkeit in Wasser | löslich |
| Löslichkeit in Sojafettsäuremethylester | > 50% |

### Beispiel 2

Ein 5000-mL-Vierhalsglaskolben mit mechanischem Rührer und aufgesetzter Destillationsvorrichtung mit Wasserabscheider und N₂-Zufuhr wurde bei 20 °C unter Stickstoffatmosphäremit 2000g (21,74 mol) Glycerin, 7,25g (0,06 mol) Phosphorsäure (85%-ig in Wasser) beschickt. Die Temperatur wurde auf 110 °C erhöht. Danach wurden innerhalb von 1 Stunde 1724g (23,91 mol) iso-Butyraldehyd mittels eines Tropftrichters zudosiert. Während der Reaktion wurde gebildetes Reaktionswasser kontinuierlich ausgekreist. Die Reaktion war nach insgesamt 3 Stunden bei 110 °C beendet. Überschüssiger iso-Butyraldehyd wurde mit einem Stickstoffstrom ausgetragen. Das Zielprodukt (Glycerinacetal von iso-Butyraldehyd) wurde in einer Menge von 3282g erhalten. Es wurde wie folgt charakterisiert:

| | |
|---|---|
| Ausbeute | 97% |
| APHA-Farbe | 5 |
| Dichte (bei 25°C) g/cm³ | 1,045 |
| Brechungsindex (bei 20°C) | 1,446 |
| Gefrierpunkt (°C) | <-50C |
| Löslichkeit in Wasser | löslich |
| Löslichkeit in Sojafettsäuremethylester | > 50% |

### Beispiel 3

Im vorliegenden Beispiel wurde im Unterschied zu den vorangehenden Beispielen kein reines Glycerin eingesetzt, sondern ein Glycerin-haltiger Rückstand, der im Zuge der Herstellung von Fettsäuremethylestern durch Umesterung von nativen Triglyceridölen mit Methanol in Gegenwart von Natriummethanolat als Katalysator angefallen war. Dieser Glycerin-haltige Rückstand, der außer Glycerin Wasser, etwas Methanol und den eingesetzten Natriummethylat-Katalysator enthielt, wird nachfolgend als Rohglycerin bezeichnet. Das Rohglycerin wies einen Glyceringehalt von 80 Gew.-% auf. Bedingt durch das im Rohglycerin enthaltene Nariummethanolat wurde (siehe unten) eine vergleichsweise erhöhte Menge an Phosphorsäure eingesetzt, da ein Teil der Phosphorsäure zunächst durch vorhandenes Natriummethanolat neutralisiert wurde.

Die Acetalbildung wurde wie folgt durchgeführt: Ein 3000-mL-Vierhalsglaskolben mit mechanischem Rührer und aufgesetzter Destillationsvorrichtung mit Wasserabscheider und N₂-Zufuhr wurde bei 20 °C unter Stickstoffatmosphäre mit 1111g (9,74 mol) Rohglycerin, 127g (1,1 mol) Phosphorsäure (85%-ig in Wasser) beschickt. Die Temperatur wurde auf 110 °C erhöht. Danach wurden innerhalb von 1 Stunde 764g (10,6 mol) iso-Butyraldehyd mittels eines Tropftrichters zudosiert. Während der Reaktion wurde gebildetes Reaktionswasser kontinuierlich ausgekreist. Die Reaktion war nach insgesamt 3 Stunden bei 110 °C beendet. Man ließ auf 70 °C abkühlen und gab 30g (0,53 mol) Kaliumhydroxid zu zwecks Neutralisation des Phosphorsäure-Katalysators. Anschließend wurde die Reaktionsmischung filtriert, wobei 1430g Zielprodukt und 200g Rückstand erhalten wurden. Das Zielprodukt wurde anschließend einer Destillation unterworfen. Dabei wurde ein klares Endprodukt im Siedebereich von 90 bis 120 °C bei einem Druck von 650 mmHg erhalten. Es wurde wie folgt charakterisiert:

| | |
|---|---|
| Ausbeute | 100% |
| APHA-Farbe | 50 |
| Gardner-Farbe | - |
| Dichte (bei 25°C) g/cm³ | 1,049 |
| Brechungsindex (bei 20°C) | 1,448 |
| Gefrierpunkt (°C) | -61,2 |
| Löslichkeit in Wasser | löslich |
| Löslichkeit in Sojafettsäuremethylester | > 50% |

## Patentansprüche

1. Verfahren zur Herstellung von Glycerinacetalen von Aldehyden ausgewählt aus der Gruppe von Isobutyraldehyd, 2-Ethylhexylaldehyd, Furfurylaldehyd und Benzaldehyd, wobei man Glycerin mit einem oder mehreren der genannten Aldehyde in Abwesenheit von organischen Lösungsmitteln und in Gegenwart von Phosphorsäure als Katalysator umsetzt, wobei man ein molares Verhältnis von Glycerin zu Aldehyd(en) im Bereich von 1 : 1 bis 1 : 1,5 einstellt.

2. Verfahren nach Anspruch 1, wobei das eingesetzte Glycerin einen Wassergehalt von maximal 20 Gew.-% aufweist.

3. Verfahren nach Anspruch 1 oder 2, wobei man die Umsetzung bei Temperaturen im Bereich von 70 bis 130 °C durchführt.

4. Verfahren nach einem der Ansprüche 1 bis 3, wobei man den Phosphorsäure-Katalysator in einer Menge von 0,1 bis 0,8 mol-% - bezogen auf Glycerin - einsetzt.

5. Verfahren nach einem der Ansprüche 1 bis 4, wobei man das Reaktionswasser kontinuierlich aus dem Reaktionsgemisch entfernt.

6. Verfahren nach einem der Ansprüche 1 bis 4, wobei man die Reaktion unter Inertgas durchführt.

7. Verwendung der durch das Verfahren gemäß den Ansprüchen 1 bis 6 erhältlichen Glycerinacetale als Lösungsmittel, Verdünnungsmittel, Emollient, Plasticizer und Netzmittel.

8. Verwendung der durch das Verfahren gemäß den Ansprüchen 1 bis 6 erhältlichen Glycerinacetale als Ausgangsstoffe für chemische Synthesen.
